**Europäisches Patentamt**

**European Patent Office**

**Office européen des brevets**

(19)

(11) Veröffentlichungsnummer: **0 010 309**

**B2**

(12) # NEUE EUROPÄISCHE PATENTSCHRIFT

(45) Veröffentlichungstag der neuen Patentschrift:
**25.01.89**

(51) Int. Cl.⁴: **A 61 K 39/395**

(21) Anmeldenummer: **79104054.6**

(22) Anmeldetag: **19.10.79**

(54) **Mittel zur Behandlung allergischer Reaktionen.**

(30) Priorität: **25.10.78 DE 2846412**

(43) Veröffentlichungstag der Anmeldung:
**30.04.80 Patentblatt 80/9**

(45) Bekanntmachung des Hinweises auf die Patenterteilung:
**11.08.82 Patentblatt 82/32**

(45) Bekanntmachung des Hinweises auf die Entscheidung über den Einspruch:
**25.01.89 Patentblatt 89/4**

(84) Benannte Vertragsstaaten:
**AT BE CH DE FR GB IT LU NL SE**

(56) Entgegenhaltungen:
**CH-A-392 780**
**CH-A-397 151**
**DE-A-2 413 642**
**DE-A-2 610 854**
**DE-A-2 752 694**
**FR-A-1 459 501**
**US-A-2 123 198**

**MICROBIOLOGY ABSTRACTS, Band III, Section B, 1968, Referenz Nummer B6066, Seite 232 V. YAKULIS et al.: "Production of Fc fragments of IgM" & J.Immunol., 100,525-529, 1968**
**CHEMICAL ABSTRACTS, Band 67, Nr. 3, 17. Juli 1967, Zusammenfassung Nr. 9 9796e, Seite 917 Columbus, Ohio, US, J.T. SGOURIS: "The preparation of plasmin-treated immune serum globulin for intravenous use", & Vox. Sang. 13(1), 71-84 (1967)**
**CHEMICAL ABSTRACTS, Band 69, Nr. 1, 1. Juli 1968, Zusammenfassung Nr. 1716z, Columbus, Ohio, US, C.A. JANEWAY et al.: "Intravenous gamma-globulin. Metabolism of gamma-globulin fragments in normal and agammaglobulinemic persons", Seite 161 & N.Engl.J.Med. 1968, 278(17), 919-23**
**CHEMICAL ABSTRACTS, Band 66, Nr. 15, 10. April 1967, Zusammenfassung Nr. 64240y, Columbus,**

(73) Patentinhaber: **BEHRINGWERKE AKTIENGESELLSCHAFT, Postfach 1140, D-3550 Marburg/Lahn (DE)**

(72) Erfinder: **Sedlacek, Hans- Harald, Dr., Sonnenhang 3, D-3550 Marburg/Lahn (DE)**
Erfinder: **Seiler, Friedrich Robert, Dr., Oberer Eichweg 10, D-3550 Marburg/Lahn (DE)**

(74) Vertreter: **Meyer- Dulheuer, Karl- Hermann, Dr., HOECHST Aktiengesellschaft Zentrale Patentabteilung Postfach 80 03 20, D-6230 Frankfurt/Main 80 (DE)**

(56) Entgegenhaltungen: (Fortsetzung)
**Ohio, US, B.V. JAGER: "Intravenous administration of modified gamma globulin" Seite 6023 & Arch.Intern.Med. 119(1), 60-4 (1967)**
**UNLISTED DRUGS, Band 19, Nr. 6, Juni 1967 Seite 65, Absatz e, Nummer MK 860 & Arch Int.Med. 119:60, Jan. 1967**
**Redner und Markow, JAMA, Bd. 185, Nr. 9, (31. August 1963), S. 64-67**
**Levy und Osler, The Journal of Immunology, Bd. 99, Nr. 6, (1967), S. 1068-1077**
**Stanworth und Smith, Clinical Allergy, Bd. 3 (1973), S. 37-41**
**Vijay und Perelmutter, The New England Journal of Medicine, Bd. 292 (1975), S. 1079**
**Vijay and Perelmutter, Int. Archs Allergy appl. Immun., Bd. 53 (1977), S. 78-87**
**Roitt, "Leitfaden der Immunologie", Dr. Dietrich Steinkopf Verlag, Darmstadt 1977, S. 139-146**
**Ovary, Federation Proceedings, Bd. 24 (1965), S. 94-97**
**Barandun, Skvaril und Morell, Schweiz. med. Wschr., Bd. 106 (1976), S. 533-542 und 580-583 bzw. Sonderdruck, S. 1-16**
**MMW, 125 (1983), S. 289 ff.**
**Int. Immunopharm. 9, pp. 635-650 (1987)**

**Die Akte enthält technische Angaben, die nach dem Eingang der Anmeldung eingereicht wurden und die nicht in dieser Patentschrift enthalten sind.**

EP 0 010 309 B2

**Beschreibung**

Die Erfindung betrifft die Herstellung eines Mittels zur Behandlung allergischer Reaktionen, das ein Fc-Fragment eines Immunglobulins der Klasse G enthält. In der Klinik wird die Bezeichnung "allergische Reaktion" für hyperergische Krankheitsbilder verwendet, die als Folge von Reaktionen zwischen Antigen-Antikörper (Reaktion vom Soforttyp) oder zwischen Antigen und Immunzelle (Reaktion vom Spättyp) auftreten (Heilmeyer: Innere Medizin 3, Auflage: Band 2, Seite 411 - 459: Springer Verlag Berlin 1971). Allen allergischen Reaktionen vom Soforttyp ist gemeinsam, daß sie durch immunologische Bindung eines Antigens an spezifische Immunoglobuline-sogenannte Reagineausgelöst werden. Derartige Reagine gehören vorwiegend, jedoch nicht ausschließlich, zu Immunglobulinen der Klasse E (IgE). Die Antigen-Bindung erfolgt über den variablen Anteil (Fab-Teil) des Immunglobulinmoleküls. Der derzeitigen Vorstellung entsprechend bindet sich das Reagin mit seinem konstanten, nicht antigenbindenden Teil, dem Fc-Teil, an die membranständigen, sogenannten Fc-Rezeptoren bestimmter Zellen (beispielsweise Mastzellen oder basophile Granulozyten). Diese Rezeptoren sind spezifisch für das Reagin. Nach Zugabe und nach immunologischer Bindung des spezifischen Antigens an das membranständige Reagin kommt es zur Freisetzung der Mediatorstoffe aus den Mastzellen und basophilen Granulozyten. Die freigesetzten Mediatorstoffe sind wesentlich an der Auslösung der Krankheitsbilder bei allergischen Reaktionen beteiligt. So werden von basophilen Granulozyten und Mastzellen nach Kontakt mit dem spezifischen Immunglobulin der Klasse E und dem korrespondierenden Antigen beispielsweise Heparin, Histamin, Serotonin, Kinin, Slow-reacting substance A, Thrombozyenaktivierender Faktor (PAP) und ein Faktor cytophil für eosinophile Granulozyten freigesetzt, welche die klinischen Erscheinungen der Allergie auslösen,

Bei allergischen Reaktionen vom Spättyp treten spezifische Antigene mit Immunzellen in Verbindung, was ebenso das Freisetzen von Mediatoren und die Bildung von Entzündungen zur Folge hat.

In der deutschen Offenlegungsschrift 2 413 642 ist ein gegen Allergien wirksames Medikament beschrieben, das Immunglobuline der D-Klasse enthält, die mit Papain abgebaut waren. IgD ist jedoch im Serum nur in geringen Mengen enthalten, so daß der Anwendung enge Grenzen gesetzt sind.

Die derzeitigen Therapiemöglichkeiten allergischer Reaktionen sind daher beschränkt symptomatisch werden Substanzen gegeben, welche die Wirkung der Mediatoren inhibieren (z. B. Antihistaminika, Cortikosteroide, Induktion von Antikörpern gegen Histamin u.a.) oder welche immunsuppressiv wirken (Antilymphozytengobulin, Zytostatika, Cortikosteroide). Ein kausaler Therapie-Ansatz besteht in der sogenannten Desensiblisierung. Diese Desensiblisierung hat zum Ziel, unter allmählich gesteigerter Zufuhr der jeweils ursächlichen Antigene eine Toleranz des Patienten gegenüber dem Antigen zu erzeugen: Diese Toleranz kann bewirkt werden durch eine verminderte Bildung spezifischer, die allergische Reaktion auslösender Antikörper oder durch Bildung von spezifischen Antikörpern einer Klasse, welche zwar das Antigen immunologisch binden, jedoch keine allergischen Reaktionen auslösen können. Eine weitere Möglichkeit besteht in der Applikation von Immunglobulinen der Klasse IgE, welche sich zwar mit dem Fc-Rezeptor der an allergischen Reaktionen beteiligten Zelle, z. B. der Mastzelle, verbinden, welche jedoch keine immunologische Reaktion mit dem die Krankheit auslösenden Antigen eingehen. Diese letztere therapeutische Möglichkeit hat jedoch bislang keine praktische Bedeutung, da zum einen gE nur in Spuren im menschlichen Blut vorhanden ist und nur in geringsten Mengen isoliert werden kann und zum zweiten durch die Applikation des Antikörpers Allergien für andere Antigene, mit welchen der verwendete Antikörper spezifisch reagiert, übertragen werden können.

Es fehlt somit ein ursachlich wirkendes Therapeutikum für die allergischen Reaktionen des Menschen. In diesem Zusammenhang durchgeführte Untersuchungen an kleinen Versuchstieren wie Maus, Ratte oder bevorzugt Meerschweinchen sind nur sehr eingeschränkt auf den Menschen übertragbar, da die allergischen Reaktionen bei diesen Versuchstieren sich in ihrer Pathophysiologie von denjenigen des Menschen unterscheiden. Da sich Versuche am Menschen wie z. B. der dem Kliniker bekannte Prausnitz-Küstner-Versuch aufgrund der Infektionsübertragung verbieten, wurden Untersuchungen mit isolierten Affenorganen durchgeführt, da die allergischen Reaktionen beim Affen bekanntermaßen denjenigen des Menschen ähneln.

Bei derartigen Versuchen wurde nun überraschend gefunden, daß ein Fc-Fragment eines Immunglobulins der Klasse IaG, die Auslösung einer allergischen Reaktion verhindern kann.

IgG ist im Gegensatz zu dem nach dem Stand der Technik vorgeschlagenen IgD in größerer und isolierbarer Menge im Serum enthalten.

Gegenstand der Erfindung ist somit die Verwendung eines durch Spaltung eines Immunglobulins der Klasse G mittels Plasmin oder Papain erhaltenen Produkts, welches das Fc-Fragment enthält, zur Herstellung eines Arzneimittels für die Prophylaxe und Therapie allergischer Reaktionen.

Im Sinne der Erfindung sind wirksam Spaltprodukte von IgG welche das Fc-Fragment besitzen und welche gewonnen wurden durch Behandlung von IgG mit Papain (Porter: Biochem. J. 73, 119, 1959) oder Plasmin (Haupt, Heide; Klin. Wschr. 47, 270, 1969). Die Spaltung darf allerdings nicht so weit geführt werden, daß es zu einem Abbau des Fc-Teils in immunologisch nicht aktive Spaltstücke kommt.

Es wurde desweiteren gefunden, daß diese Spaltprodukte, enthaltend das Fc-Fragment von IgG in geringerem Umfang Komplement aktivieren als komplexgebundene Immunglobuline. Da die Komplementaktivierung als ein Maßstab für die Verträglichkeit eines Immunglobulinpräparates gilt, müssen Spaltprodukte von IgG als verträglicher angesehen werden als komplexgebundenes IgG. Die komplementaktivierende Wirksamkeit von komplexgebundenem IgG oder Spaltprodukten von IgG wurde im Test nach Kabat und Mayer (Experimental immunochemistry, 2. Auflage; Thomas, Sprungfield) bestimmt. Die Ergebnisse sind in Tabelle 1 aufgeführt.

**Tabelle 1**

Komplementaktivierende Wirksamkeit von komplexgebundenem IgG* oder von Spaltprodukten des IgG* (ausgedruckt in %)

| | |
|---|---|
| IgG-Aggregate (65°/30 min erwärmt) | 100 % |
| IgG-Aggregate (Kaprylsäure und Ammoniumsulfatpräzipitaton) | 100 % |
| IgG-Immunkomplexe | 100 % |
| Papain Fc-Fragmente | 30 % |
| IgG, sulfoniert | 0 % |
| F(ab)$_2$-Fragmente von IgG | 0 % |

* jeweils 1 %-ige Proteinlösungen

Zur Therapie kann das erfindungsgemäße Präparat lokal (d.h. auf Haut oder Schleimhäute) oder systemisch appliziert werden. Bei systemischer Applikation ist die parenterale Applikation (beispielsweise i.m. oder s.c.) einer oralen vorzuziehen. Zur lokalen oder parenteralen Applikation ist das erfindungsge- mäße Präparat in einer dem Fachmann bekannten Formulierung zuzubereiten. Jedoch kann zur Applikation auch eine Vermischung mit Serum oder mit Serumbestandteilen erfolgen. Bei oraler Applikation muß das erfindungsgemäße Präparat in einer dem Fachmann bekannten Formulierung gegeben werden, welche eine Verdauung des Proteins im Magen und erstem Dünndarmabschnit verhindert.

Die Applikation des erfindungsgemäßen Präparates kann einfach oder mehrfach in einer Dosierung zwischen 10 ng/kg Körpergewicht (KG) bis 1 g/kg KG erfolgen.

Die Erfindung ist in nachstehenden Beispielen näher erläutert.

**Beispiel 1**

Affen (Cynomolgen) wurde in Narkose die Bauchhöhle eröffnet. Ein bestimmter Dünndarmabschnitt (Ileum) wurde entnommen und sofort in körperwarme Nährlösung (dem Fachmann als Tyrodelösung bekannt) gegeben. Dünne ca. einen halben cm breite und 2 cm lange Längsstreifen des Darmes wurden in einer körperwarm gehaltenen, von Sauerstoff durchperlten Tyrodelösung zwischen zwei Haken derart eingespannt, daß Muskelkontraktionen des Darmabschnittes über einen Registriermechanismus aufgezeichnet werden konnten (Schultze-Dale-Apparat). Sodann wurde ein Darmabschnitt mit einem nach Papainspaltung erhaltenen Fc-Fragment von IgG (Endkonzentration im organbad 35 ng/ml), ein anderer Darmabschnitt zur Kontrolle mit physiologischer Kochsalzlösung oder mit Albumin oder mit Fab$_2$-Fragmenten von IgG, hergestellt nach Reid (Immunology 20, 649, 1971) in entsprechender Endkonzentration für ca. 30 min bei 37° inkubiert. Nachfolgend wird jedem der Ansätze Reagin-haltiges Patientenserum, dem das Allergie auslösende Antigen zugesetzt worden war, in einer Menge von 0,2 ml und 0,5 ml auf 30 ml Organbadlösung zugesetzt. Am nicht vorbehandelten Darm führt die Applikation des Gemisches von Serum und Antigen zu allergischen Reaktionen, d.h. zu Erhöhung des Tonus und der Frequenz der Darmmobilität. Wie aus der Tabelle 2 ersichtlich, hat jedoch die Vorinkubation mit Papain-Fc einen hemmenden Einfluß auf die durch das Gemisch aus reginhaltigem Patientenserum und Allergie auslösendem Antigen verursachte allergische Reaktion.

**Tabelle 2**

Hemmung der allergischen Reaktion am Affendarm durch Fc-Fragmente von IgG
Reagin : Serum von Patienten, allergisch gegen Schistosoma-Antigen

| Vorinkubation des Darmes mit | Anstieg des Darmtonus und der Kontraktions-frequenz (in %) (im Vergleich zu 3 µg Histamin/ml = 100 %) | | Hemmung der allergischen Reaktion (in %) | |
|---|---|---|---|---|
| | 7 µl/ml* | 17 µl/ml* | 7 µl/ml* | 17 µl/ml* |
| - | 18 | 30 | - | - |
| Papain-Fc-Fragmenten | 0 | 20 | 100 | 33 |

(*) Allergische Reaktion, ausgelöst durch Zugabe von 7 µl bzw. 17 µl/ml eines Gemisches von Allergikerserum und Schistosoma-Antigen
(v : v = 1 : 1).

**Beispiel 2**

Längsstreiten vom Affendarm, gewonnen wie im Beispiel 1 erwähnt, werden mit verschiedenartig hergestellten Fc-Fragmenten von IgG (z. B. Papain-Fc) sowie mit auf unterschiedliche Art (Erhitzen, Kaprylsäure-Präzipitaton oder Ammoniumsulfat-Präzipitation) komplexgebundenem d.h. aggregiertem IgG und zur Kontrolle mit F(ab)₂-Fragmenten von IgG, oder mit chemisch modifiziertem Papain-Fc (über eine Diazotierung chemisch verändertes Fc gemäß US Pat. 3 873 697) in einer Endkonzentration von 500 µg/ml Tyrodelösung für 15 min bei 37°C inkubiert. Nachfolgend werden die Organstücke entnommen und bei 37°C in einer Tyrodelösung, enthaltend 50 µl/ml Patientenserum inkubiert. Nach 15 min werden die Organstreifen wie im Beispiel 1 erwähnt in die Schultz-Dale-Apparatur eingehängt und 0,5 ml Kuhhaarsuspension (20 mg Kuhhaare, kleingeschnitten und suspendiert in 10 ml Tyrodelösung) dem Organbad (30 ml) hinzugegeben. Die Ergebnisse sind in Tabelle 3 aufgeführt. Sie zeigen deutlich eine vollständige Hemmung der allergischen Reaktion durch Immunologisch verändertes Fc, d.h. durch komplexgebundenes (aggregiertes) IgG und durch Fc nach Papain-spaltung, wesentlich geringer durch Fc nach Plasmin-spaltung, jedoch keine Hemmung durch F(ab)₂-Fragmente von IgG, und durch chemisch verändertes Fc.

**Tabelle 3**

Hemmung der allergischen Reaktion am Affendarm durch Fc-Fragmente bzw. Aggregate (Prüfpräparate) von IgG

| Vorinkubation des Darmes mit (Prüfsubstanzen) | Allergen * | Reagin : Serum eines Patienten, allergisch gegen Kuhhaare und (geringer) Mäusehaare — Anstieg des Darmtonus und der Konzentrationsfrequenz (in %) (im Vergleich zu 3 µg Histamin/ml = 100 %) | Hemmung der allergischen Reaktion |
|---|---|---|---|
| - | Kuhhaare | 36 | |
| - | Mäusehaare | 20 | |
| - | Rattenhaare | 0 | |
| F(ab)$_2$-Fragm. | Kuhhaare | 37 | 0 |
| Aggregate (56°/10 min erwärmt) | Kuhhaare | 0 | 100 |
| Papain-Fc-Frag. | Kuhhaare | 0 | 100 |
| Plasmin-Fc-Frag. | Kuhhaare | 18 | 50 |
| chem. behand. Papain-Fc-Frag. | Kuhhaare | 37,5 | -0 |
| IgG-Aggregate (65° C/30 min erwärmt) | Kuhhaare | 0 | 100 |
| IgG (Aggregatanteil 20 %) (Kaprylsäure und Ammoniumsulfat-Präzipitation) | Kuhhaare | 0 | 100 |
| IgG (Aggregatanteil 10 %) (Kaprylsäure und Ammoniumsulfat-Präzipitation) | Kuhhaare | 6 | 84 |

* Zugabe des Allergens nach Vorinkubation des Darmes mit Prüfsubstanzen, und mit dem Allergikerserum (17 µl/ml).

**Beispiel 3** (Patient, allergisch gegen Kaninchenhaare)

Experimenteller Ansatz wie in Beispiel 2: Inkubation der Längsstreifen vom Affendarm mit verschiedenen Fc-Fragmenten von IgG (Papain-Fc, Plasmin-Fc) bzw. mit auf unterschiedliche Art komplexgebundenem IgG (Anlagen-Immunkomplexe im Antigen und im Antikörperüberschuß, hitzebehandeltes IgG, Kapryl- und Ammoniumsulfat-behandeltes IgG gemäß Schutze, Heremans 1966). Zur Kontrolle Inkubation mit F(ab)$_2$-Fragmenten von IgG, dessen Fc-Teil chemisch modifiziert wurde (Sulfonylierung nach Schutze und Heremans, Molecular Biology of Human Proteins, S. 43, Elsevier Amsterdam 1966) und mit IgG, durch Ultrazentrifugation (2 Stunden bei 120 000 g) von Aggregaten freigemacht.

Nachfolgend Inkubation mit dem Patientenserum. Danach Einhängen des Organes in die Schultz-Dale-Apparatur und Zugabe von Kaninchen-haarsuspension.

Die Ergebnisse zeigen eine vollständige Hemmung der allergischen Reaktion durch komplexgebundenes IgG und Fc nach Papain- und Plasmin-Spaltung, jedoch keine Hemmung durch F(ab)$_2$-Fragmente von IgG, durch im Fc-Teil chemisch verändertes IgG und durch Aggregat-freies IgG.

**Beispiel 4** (Patient, allergisch gegen Pollen)

Patient H.S. mit ausgeprägter, seit 5 Jahren bestehender Pollenallergie. Zu Beginn der Erkrankung bei jahreszeitlich bedingtem Pollenflug intranasale Applikation von 0,5 ml Papain-Fc verteilt auf beide Nasenlöcher (10 mg/ml phys. NaCl-Lösung). Wiederholung der Behandlung viermal im Abstand von 2 Wochen. Ergebnis: Starke Verringerung des Niesreizes, und Sekretflusses der Nase bis zur Symtomlösigkeit.

**Patentansprüche**

1. Verwendung eines durch Spaltung eines Immunglobulins der Klasse G mittels Plasmin erhaltenen Produkts, das Fc-Fragment enthält zur Herstellung eines Arzneimittels für die Prophylaxe und Therapie allergischer Reaktionen.

2. Verwendung eines durch Spaltung eines Imunglobulins der Klasse G mittels Papain erhaltenen Produkts, welches das Fc-Fragment enthält, zur Herstellung eines Arzneimittels für die Prophylaxe und Therapie allergischer Reaktionen.

**Claims**

1. The use of a product prepared by cleaving an immunoglobulin of class G by means of plasmin and containing the Fc fragment in a process for preparing a medicament for prophylaxis and therapy of allergic reactions.

2. The use of a product prepared by cleaving an immunoglobulin of class G by means of papain and containing the Fc fragment in a process for preparing a medicament for prophylaxis and therapy of allergic reactions.

**Revendications**

1. Utilisation d'un produit obtenu par scission d'une immunoglobuline de la classe G au moyen de plasmine, qui contient un fragment Fc, pour la préparation d'un médicament pour la prophylaxie et le traitement de réactions allergiques.

2. Utilisation d'un produit obtenu par scission d'une immunoglobuline de la classe G au moyen de papaïne, qui contient le fragment Fc, pour la préparation d'un médicament pour la prophylaxie et le traitement de réactions allergiques.